# EUROPEAN PATENT SPECIFICATION

(11) **EP 0 783 521 B1**
(45) Date of publication and mention of the grant of the patent: **09.10.2002**
(21) Application number: 95932735.4
(22) Date of filing: 13.09.1995
(51) Int. Cl.: C07K 14/60, C07K 5/027, C07K 5/117, C07K 5/103, A61K 38/25, A61K 38/07, A61K 38/06

(54) **POLYPEPTIDE COMPOUNDS CONTAINING D-2-ALKYLTRYPTOPHAN CAPABLE OF PROMOTING THE RELEASE OF GROWTH HORMONE**
D-2-ALKYLTRYPTOPHAN ENTHALTENDE POLYPEPTIDE MIT WACHSTUMHORMON FREISETZENDER AKTIVITAET
COMPOSES POLYPEPTIDES CONTENANT D-2-ALKYLTRYPTOPHANNE POUVANT PROMOUVOIR LA LIBERATION DE L'HORMONE DE CROISSANCE

(30) Priority: 27.09.1994 IT MI941954; 16.06.1995 IT MI951293
(43) Date of publication of application: 16.07.1997
(73) Proprietor: Ardana Bioscience Limited, Edinburgh EH2 3NS (GB)
(72) Inventor: Ardana Bioscience Limited, Edinburgh EH2 3NS (GB)
(74) Representative: Miles, John Stephen
(86) International application number: PCT/EP95/03601
(87) International publication number: WO 96/010040

(56) References cited:
- EP-A- 0 083 864
- WO-A-88/09780
- WO-A-91/18016
- WO-A-94/07519
- LIFE SCIENCES, vol. 54, no. 18, 1994 pages 1321-1328, R.DEGHENGI ET AL 'GH-RELEASING ACTIVITY OF HEXARELIN, A NEW GROWTH HORMONE RELEASING PEPTIDE, IN INFANT AND ADULT RATS' cited in the application
- ENDOCRINOLOGY vol. 128, no. 4, 1991, pages 2027 - 2035
- ENDOCRINOLOGY vol. 114, no. 5, 1984, page 1531

## Description

The present invention relates to oligopeptide compounds containing a D-2-alkyltryptophan amino acid and which are capable of releasing growth hormone (GH) from somatotropes, and are active by oral route.

### Background of the invention

The increase of growth hormone (GH) levels in mammals after the administration of compounds inducing GH release can yield to growth acceleration and muscular mass increase and enhanced production of milk, if sufficiently high GH levels are obtained after the administration. Moreover, it is known that the increase of growth hormone levels in mammals can be achieved by administering known growth hormone release agents, such as growth hormone release hormones (GHRH).

The increase of growth hormone levels in mammals can also be obtained by administering growth hormone release peptides, some of them having previously been described, for example in US 4,223,019, US 4,223,020, US 4,223,021, US 4,224,316, US 4,226,857, US 4,228,155, US 4,228,156, US 4,228,157, US 4,228,158, US 4,410,512, US 4,410,513, US 4,411,890 and US 4,839,344.

Therefore, rather simple short chain-polypeptides, capable of promoting growth hormone release on condition that they are easily and conveniently preparable, as well as of easy purification and formulation and active when administered by oral route, are presently desired.

One of the more studied growth hormone release peptides (GHRP) has been known since many years as GHRP-6 (C.Y. Bowers et al., Endocrinology 114:1537 (1984) and has the formula His-D-Trp-Ala-Trp-D-Phe-Lys-NH₂. GHRP-6 releases growth hormone in vitro and in vivo and is orally active in animals and in humans. Its molecular mechanism has been studied, as well as the one of its analogue heptapeptide GHRP-1 (Cheng et al., Endocrinology 124:2791 (1989); M.S. Akman et al., Endocrinology 132:1286 (1993)). It was found that, contrarily to natural GHRH, these peptides act through different receptors for the release of GH and through a different mechanism, which is independent from cAMP and which operates through other intracellular pathways, such as the mobilization of calcium supplies and a proteinkinase C (PKC)-dependent process (L. Bresson-Bépoldin and L. Dufy-Barbe, Cell. Calcium 15, 247, (1994).

### Summary of the invention

In a completely surprising manner it has now been found that the introduction of a D-2-alkyltryptophan (2-Mrp) in the oligopeptide of the GHRP series, modifies the known intracellular mechanism of GH release. In some cases, it has been evidenced a substantial increase of the activity of the adenylcyclase in the anterior pituitary glands, both of murine origin, and of human origin, but the specific mechanism remains still to be completely clarified.

Thus, the modification given by a single alkyl group in the 2-position of tryptophan (in its D-conformation), besides favourably increasing the stability of the Trp residue (R. Deghenghi WO 91/18016 published on 28.11.1991 and R. Deghenghi et. al, Life Sciences 54, 1321, (1994)), is responsible for a completely unexpected change of the intracellular mechanisms which now are independent from calcium, sometimes dependent on adenylcyclase and more similar to those of GHRH, as well as other peptide hormones. (James D. Watson et al., Molecular Biology of the Gene, 4^{th} ed., The Benjamin /Cummings Publishing Company, Inc., Menlo Park, California, 1987, pag. 60).

Another unexpected characteristic feature of the present invention is the very high potency of some penta-, hexa-, and heptapeptides and the favourable oral activity/potency ratio also of the smaller tetrapeptides of the series.

The oligopeptides of the present invention are selected from the group consisting of:
IMA-D-Mrp-D-Trp-Phe-LysNH₂;
IMA-D-Mrp-D-β-Nal-Phe-LysNH₂;
INIP-D-Mrp-D-Trp-Phe-LysNH₂;
INIP-D-Mrp-D-β-Nal-Phe-LysNH₂;
GAB-D-Mrp-D-Trp-Phe-LysNH₂;
GAB-D-Mrp-D-β-Nal-Phe-LysNH₂;
GAB-D-Mrp-D-β-Nal-NH₂;
GAB-D-Mrp-D-β-Nal-OC₂H₅;
4-(aminomethyl)cyclohexanecarbonyl-D-Mrp-D-Trp-Phe-Lys-NH₂;
4-(aminomethyl)cyclohexanecarbonyl-D-Mrp-D-β-Nal-Phe-Lys-NH₂.
Imidazolacetyl-D-Mrp-Ala-Trp-D-Phe-LysNH₂;
D-Ala-D-Mrp-Ala-Trp-D-Phe-LysNH₂;
D-Thr-D-Mrp-Ala-Trp-D-Phe-LysNH₂;
His-D-Mrp-Ala-TrpNH₂;
D-Thr-D-Mrp-Ala-TrpNH₂;
His-D-Mrp-Ala-Phe-D-Trp-LysNH₂;
D-Thr-D-Mrp-Ala-Trp-D-Phe-Lys-D-ThrNH₂;
D-Thr-His-D-Mrp-Ala-Trp-D-Phe-LysNH₂;
His-D-Mrp-Ala-Trp-D-Phe-Lys-ThrNH₂;
His-D-Mrp-Ala-Trp-D-Phe-Lys-D-ThrNH₂,
and their addition salts with pharmaceutically acceptable organic or inorganic acids, where Mrp is 2-(C₁-C₃)alkyltryptophan, and
- Gly =: Glycine
- Tyr =: L-Tyrosine
- Ile =: L-Isoleucine
- Glu =: L-Glutamic Acid
- Thr =: L-Threonine
- Phe =: L-Phenylalanine
- Ala =: L-Alanine
- Lys =: L-Lysine
- Asp =: L-Aspartic Acid
- Cys =: L-Cysteine
- Arg =: L-Arginine
- Gln =: L-Glutamine
- Pro =: L-Proline
- Leu =: L-Leucine
- Met =: L-Methionine
- Ser =: L-Serine
- Asn =: L-Asparagine
- His =: L-Histidine
- Trp =: L-Tryptophan
- Val =: L-Valine.
Moreover,
- D-β-Nal =: D-β-Naphthylalanine;
- INIP =: Isonipecotyl;
- IMA =: Imidazolylacetyl;
- GAB =: 4-aminobutyryl;
- Mrp =: 2-alkyltryptophan.

According to the present invention, for alkyl it is intended lower alkyl, comprising from 1 to 3 carbon atoms. Examples of lower alkyl are methyl, ethyl, propyl, isopropyl. Among these methyl group is mostly preferred.

All the three letter-abbreviations of the amino acids preceded by a "D" indicate the D-configuration of the aminoacidic residue.

These compounds can be administered parenterally, but more conveniently intranasally and orally, or can be formulated in controlled release systems, such as biodegradable microcapsules, microspheres, subcutaneous implants and the like.

The polypeptide compounds according to the present invention can be synthesized according to the usual methods of peptide chemistry, both solid-phase and solution, or by means of the classical methods known in the art. The solid-phase synthesis starts from C-terminal end of peptide. A suitable starting material can be prepared, for example attaching the required protected alpha-amino acid to a chloromethylated resin, a hydroxymethylated resin, a benzhydrylamine resin (BHA), or to a para-methylbenzhydrylamine resin (p-Me-BHA). As example, a chloromethylated resin is sold with the Trade Mark BIOBEADS (R) SX 1 by BioRad Laboratories, Richmond, California. The preparation of the hydroxymethyl resin is described by Bodansky et al., Chem. Ind. (London) 38, 15997, (1966). The BHA resin is described by Pietta and Marshall, Chem. Comm., 650 (1970) and is commercially available by Peninsula Laboratories Inc., Belmont, California.

After the starting attachment, the protecting group of the alpha-amino acid can be removed by means of different acid reagents, comprising trifluoroacetic acid (TFA) or hydrochloric acid (HCl) dissolved in organic solvents at room temperature. After the removal of the protecting group of the alpha-amino acid, the remaining protected amino acids can be coupled step by step in the desired order. Each protected amino acid can generally be reacted in excess of about three times using a suitable carboxyl activating group, such as dicyclohexylcarbodiimiide (DCC) or diisopropylcarbodiimide (DIC) dissolved, for example, in methylene chloride (CH₂Cl₂) or dimethylformamide (DMF) and their mixtures. After the desired aminoacidic sequence has been completed, the desired peptide can be cleaved from the supporting resin by treatment with a reagent such as hydrogen fluoride (HF), which not only cleaves the peptide from the resin, but also the more common protecting groups of the lateral chains. When a chloromethylated resin or a hydroxymethylated resin is used, the treatment with HF leads to the formation of the acid peptide in free form. When a BHA or p-Me-BHA resin is used, the treatment with HF directly leads to the formation of the amide peptide in free form.

The above discussed solid-phase procedure is known in the art and was described by Atherton and Sheppard, Solid Phase Peptide Synthesis (IRL Press, Oxford, 1989).

Some methods in solution, which can be used to synthesize the peptide moieties of the present invention are detailed in Bodansky et al., Peptide Synthesis, 2nd edition, John Wiley & Sons, New York, N.Y. 1976 and from Jones, The Chemical Synthesis of Peptides, (Clarendon Press, Oxford, 1994).

These compounds can be administered to animals and humans at an effective dose which can be easily determined by the expert in the field and which can vary according to the specie, age, sex and weight of the treated subject. For example, in humans, when intravenously administered, the preferred dose falls in the range from about 0.1 µg to 10 µg of total peptide per kg of body weight. When orally administered, typically higher amounts are necessary. For example, in humans for the oral administration, the dosage level is typically from about 30 µg to about 1000 µg of polypeptide per kg of body weight. The exact level can be easily determined empirically based on the above disclosure.

Compositions, which comprise as active ingredient the organic and inorganic addition salts of the above described polypeptides and their combinations, optionally, in admixture with a vehicle, diluent, matrix or delayed release coating, are also comprised in the scope of the present invention. The delayed release pharmaceutical forms, comprising bioerodible matrixes, suitable for subcutaneous implant are particularly interesting. Examples of these matrices are described in WO9222600 and WO9512629.

### Biological activity

In vivo activity of these compounds was determined in ten day-rats, which were subcutaneously injected (s.c.) with a dose of 300 µg/kg or with different doses in dose-response studies, according to what described in detail by R. Deghenghi et. al, Life Sciences 54, 1321, (1994). The results are resumed in the Table below, the released GH was measured after 15 minutes from the treatment.

The GHRP-2 (reference standard) has the structure D-Ala-D-β-Nal-Ala-Trp-D-Phe-Lys-NH₂ (Chen and Clarke, J. Neuroend. 7, 179 (1995).

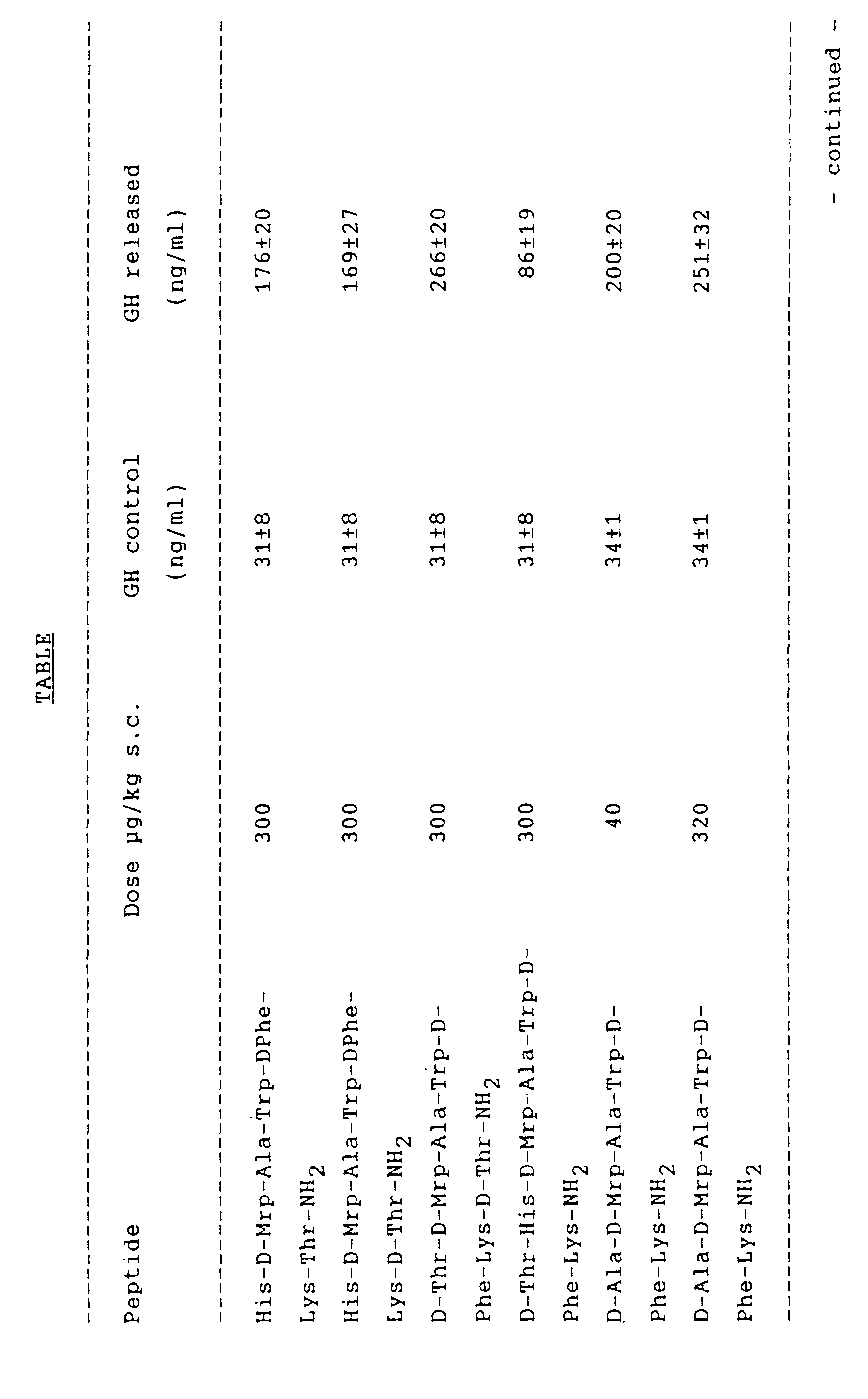

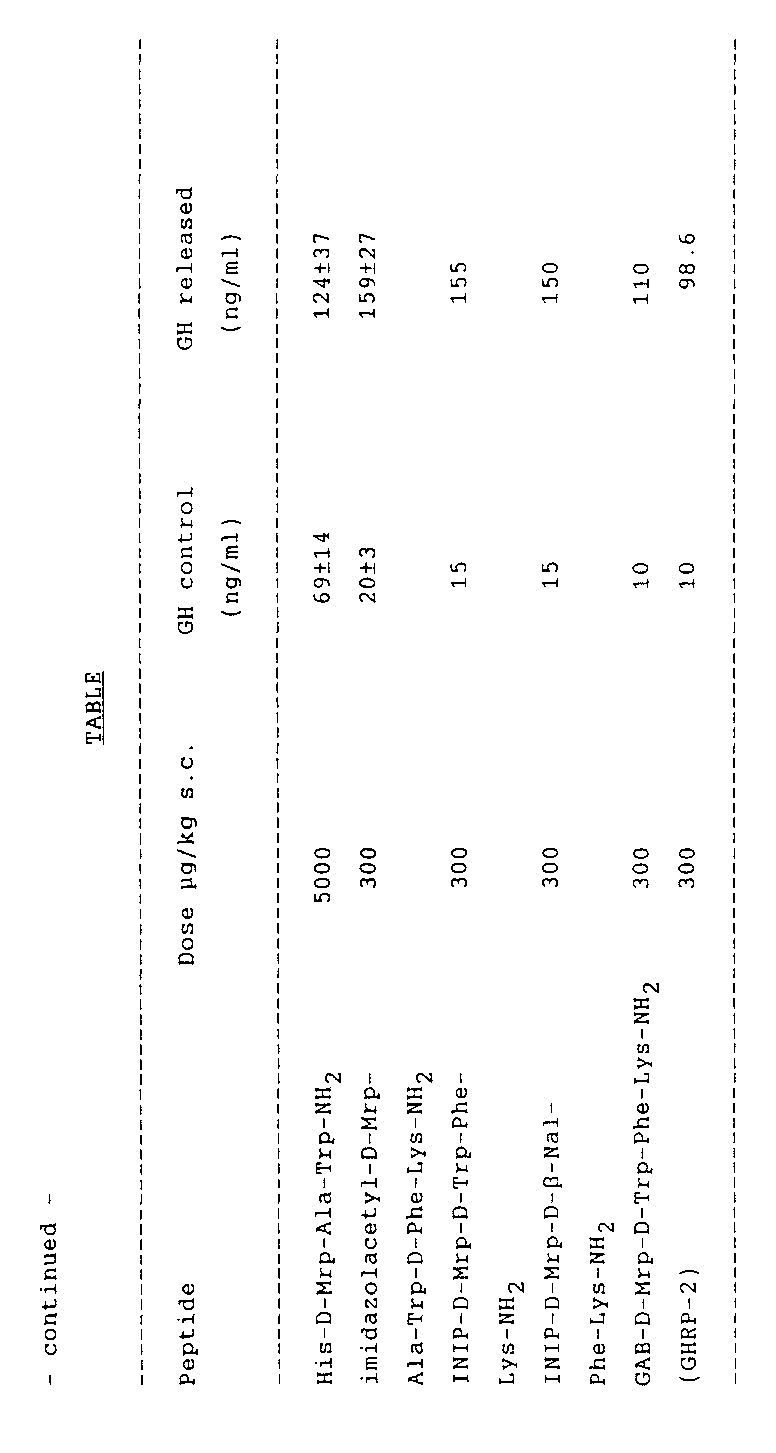

In vitro activity on adenylcyclase activity was measured in anterior pituitary gland cells from rats weighing 150 g and showed a 30% increase compared with the baseline with EC₅₀=0.23 nm for the peptide D-Ala-D-Mrp-Ala-Trp-D-Phe-LysNH₂, whereas GHRP-6 (His-D-Trp-Ala-Trp-D-Phe-LysNH₂) resulted inactive.

The following examples further illustrate the invention.

### EXAMPLE 1

### Synthesis of GAB-D-Mrp-D-Trp-Phe-Lys-NH₂ (Mrp= 2-methylthryptophan).

The synthesis was carried out by solid-phase with 9-fluorenylmethyloxycarbonyl (Fmoc)-aminoacids involving resin preparation, assembly in a reactor column according to one of several methods available to the skilled in the art, as exemplified in "Solid phase peptide synthesis" by E.Atherton and R.C. Sheppard, IRL press at Oxford University press, 1989. The protected amino acids are Fmoc-Lys(Fmoc)-Opfp (Opfp=pentafluorophenyl ester), Fmoc-Phe-Opfp, Fmoc-D-Trp-Opfp, Fmoc-D-2-MeTrp-Opfp and Fmoc-GAB-Opfp (GAB=gamma-aminobutyric). Alternatively, the use of Castro's reagents, BOP and PyBOP (cfr. Le Nguyen and Castro (1988) in Peptide Chemistry 1987, p.231-238. Protein Research Foundation Osaka; and Tetrahedron Letters 31,205 (1990) can also be used advantageously as direct coupling reagents.

After cleavage and isolation, the title peptide was purified as acetate salt. Purity (HPLC): 98%, MW (M+H⁺)=764.3 (theoretical=763.9).

### EXAMPLE 2

According to the procedures described in Example 1, the following peptides were prepared and obtained as acetate salts:
INIP-D-Mrp-D-Trp-Phe-Lys-NH₂; purity (HPLC)=99.0%, MW (M+H⁺=790.4; theoretical=790.0)
INIP-D-Mrp-D-β-Nal-Phe-Lys-NH₂; purity (HPLC)=96.5%, MW (M+H⁺=801.4; theoretical=801.0)
IMA-D-Mrp-D-Trp-Phe-Lys-NH₂; purity (HPLC)=99.2%, MW (M+H⁺=786.5; theoretical=786.8)
IMA-D-Mrp-D-β-Nal-Phe-Lys-NH₂; purity (HPLC)=97.3%, MW (M+H+=798.3; theoretical=797.9)
GAB-D-Mrp-D-β-Nal-Phe-Lys-NH₂;
4-(aminomethylcyclohexanecarbonyl-D-Mrp-D-Trp-Phe-Lys-NH₂;
4-(aminomethyl)ciclohexanecarbonyl-D-Mrp-D-β-Nal-Phe-Lys-NH₂;
D-Ala-D-Mrp-Ala-Trp-D-Phe-LysNH₂;
D-Thr-D-Mrp-Ala-Trp-D-Phe-LysNH₂;
His-D-Mrp-Ala-Phe-D-Trp-LysNH₂;
Tyr-His-D-Mrp-Ala-Trp-D-Phe-LysNH₂;
His-D-Mrp-Ala-TrpNH₂;
D-Thr-D-Mrp-Ala-TrpNH₂;
wherein Mrp is 2-methyltryptophan, INIP, IMA and GAB are as defined above.

## Claims

1. A peptide selected from the group consisting of:
IMA-D-Mrp-D-Trp-Phe-LysNH₂;
IMA-D-Mrp-D-β-Nal-Phe-LysNH₂;
INIP-D-Mrp-D-Trp-Phe-LysNH₂;
INIP-D-Mrp-D-β-Nal-Phe-LysNH₂;
GAB-D-Mrp-D-Trp-Phe-LysNH₂;
GAB-D-Mrp-D-β-Nal-Phe-LysNH₂;
GAB-D-Mrp-D-β-Nal-NH₂;
GAB-D-Mrp-D-β-Nal-OC₂H₅;
4-(aminomethyl)cyclohexanecarbonyl-D-Mrp-D-Trp-Phe-Lys-NH₂;
4-(aminomethyl)cyclohexanecarbonyl-D-Mrp-D-β-Nal-Phe-Lys-NH₂.
Imidazolacetyl-D-Mrp-Ala-Trp-D-Phe-LysNH₂;
D-Ala-D-Mrp-Ala-Trp-D-Phe-LysNH₂;
D-Thr-D-Mrp-Ala-Trp-D-Phe-LysNH₂;
His-D-Mrp-Ala-TrpNH₂;
D-Thr-D-Mrp-Ala-TrpNH₂;
His-D-Mrp-Ala-Phe-D-Trp-LysNH₂;
D-Thr-D-Mrp-Ala-Trp-D-Phe-Lys-D-ThrNH₂;
D-Thr-His-D-Mrp-Ala-Trp-D-Phe-LysNH₂;
His-D-Mrp-Ala-Trp-D-Phe-Lys-ThrNH₂;
His-D-Mrp-Ala-Trp-D-Phe-Lys-D-ThrNH₂,
where Mrp is 2-(C₁-C₃)alkyltryptophan, and the addition salts thereof with pharmaceutically acceptable organic or inorganic acids.

2. A peptide according to claim 1, wherein Mrp is 2-methyltryptophan.

3. The use of the peptides of claims 1-2 for the manufacturing of a medicament useful for promoting the release of growth hormone in an animal.

4. The use according to claim 3, wherein the medicament is useful in human medicine.

5. The use of the peptides of claim 1 for the manufacturing of a medicament useful to promote the increase of the intracellular activity of adenylcyclase.

6. Pharmaceutical compositions comprising an effective amount of at least a peptide of claim 1 as active ingredient, optionally in admixture with carriers and excipients.

7. Compositions according to claim 6 for parenteral, intranasal, oral, controlled release administration or subcutaneous implants.

## Patentansprüche

1. Peptid, das ausgewählt ist aus der Gruppe von:
IMA-D-Mrp-D-Trp-Phe-LysNH₂,
IMA-D-Mrp-D-β-Nal-Phe-LysNH₂,
INIP-D-Mrp-D-Trp-Phe-LysNH₂,
INIP-D-Mrp-D-β-Nal-Phe-LysNH₂,
GAB-D-Mrp-D-Trp-Phe-LysNH₂,
GAB-D-Mrp-D-β-Nal-Phe-LysNH₂,
GAB-D-Mrp-D-β-Nal-NH₂,
GAB-D-Mrp-D-β-Nal-OC₂H₅,
4-(Aminomethyl) cyclohexancarbonyl-D-Mrp-D-Trp-Phe-LysNH₂,
4-(Aminomethyl) cyclohexancarbonyl-D-Mrp-D-β-Nal-Phe-LysNH₂, Imidazolacetyl-D-Mrp-Ala-Trp-D-Phe-LysNH₂,
D-Ala-D-Mrp-Ala-Trp-D-Phe-LysNH₂,
D-Thr-D-Mrp-Ala-Trp-D-Phe-LysNH₂,
His-D-Mrp-Ala-TrpNH₂,
D-Thr-D-Mrp-Ala-TrpNH₂,
His-D-Mrp-Ala-Phe-D-Trp-LysNH₂,
D-Thr-D-Mrp-Ala-Trp-D-Phe-Lys-D-ThrNH₂,
D-Thr-His-D-Mrp-Ala-Trp-D-Phe-LysNH₂,
His-D-Mrp-Ala-Trp-D-Phe-Lys-ThrNH₂,
His-D-Mrp-Ala-Trp-D-Phe-Lys-D-ThrNH₂,
worin Mrp 2-(C₁-C₃)Alkyltryptophan ist, und die Säureadditionssalze desselben mit pharmazeutisch akzeptablen organischen oder anorganischen säuren.

2. Peptid nach Anspruch 1, worin Mrp 2-Methyltryptophan ist.

3. Verwendung der Peptide nach Anspruch 1 - 2 zur Herstellung eines Medikaments, das zur Förderung der Freisetzung eines Wachstumshormons in einem Lebewesen verwendbar ist.

4. Verwendung nach Anspruch 3, wobei das Medikament in der Humanmedizin verwendbar ist.

5. Verwendung der Peptide nach Anspruch 1 zur Herstellung eines Medikaments, das zur Förderung der Erhöhung der intrazellulären Aktivität von Adenylcyclase verwendbar ist.

6. Pharmazeutische Zusammensetzungen, die eine wirksame Menge von mindestens einem Peptid nach Anspruch 1 als Wirkstoff optional im Gemisch mit Trägern und Streckmitteln umfassen.

7. Zusammensetzungen nach Anspruch 6 zur parenteralen, intranasalen, oralen Verabreichung mit gesteuerter Freisetzung oder für subkutane Implantate.

## Revendications

1. Peptide choisi dans un groupe comprenant du :
IMA-D-Mrp-D-Trp-Phe-LysNH₂ ;
IMA-D-Mrp-D-β-Nal-Phe-LysNH₂ ;
INIP-D-Mrp-D-Trp-Phe-LysNH₂ ;
INIP-D-Mrp-D-β-Nal-Phe-LysNH₂ ;
GAB-D-Mrp-D-Trp-Phe-LysNH₂ ;
GAB-D-Mrp-D-β-Nal-Phe-LysNH₂ ;
GAB-D-Mrp-D-β-Nal-NH₂ ;
GAB-D-Mrp-D-β-Nal-OC₂H₅ ;
4-(aminométhyl)cyclohexanecarbonyl-D-Mrp-D-Trp-Phe-LysNH₂ ;
4-(aminométhyl)cyclohexanecarbanyl-D-Mrp-D-β-Nal-Phe-LysNH₂ ;
Imidazolacétyl-D-Mrp-Ala-Trp-D-Phe-LysNH₂ ;
D-Ala-D-Mrp-Ala-Trp-D-Phe-LysNH₂ ;
D-Thr-D-Mrp-Ala-Trp-D-Phe-LysNH₂ ;
His-D-Mrp-Ala-TrpNH₂ ;
D-Thr-D-Mrp-Ala-TrpNH₂ ;
His-D-Mrp-Ala-Phe-D-Trp-LysNH₂ ;
D-Thr-D-Mrp-Ala-Trp-D-Phe-Lys-D-ThrNH₂ ;
D-Thr-His-D-Mrp-Ala-Trp-D-Phe-LysNH₂ ;
His-D-Mrp-Ala-Trp-D-Phe-Lys-ThrNH₂ ;
His-D-Mrp-Ala-Trp-D-Phe-Lys-D-ThrNH₂ ;
où Mrp est le 2-(C₁-C₃)alkyltryptophane, et l'ajout des sels correspondants avec des acides organiques ou inorganiques acceptables pour des applications pharmaceutiques.

2. Peptide selon la revendication 1, dans lequel Mrp est le 2-méthyltryptophane.

3. Utilisation des peptides selon les revendications 1 et 2 pour la fabrication d'un médicament utilisé pour favoriser la libération d'hormones de croissance chez les animaux.

4. Utilisation selon la revendication 3, dans laquelle le médicament est utile en médecine humaine.

5. Utilisation des peptides selon la revendication 1 pour la fabrication d'un médicament utile pour favoriser l'augmentation de l'activité intracellulaire de l'adénylcyclase.

6. Compositions pharmaceutiques comprenant une quantité efficace d'au moins un peptide selon la revendication 1 comme ingrédient actif, de façon optionnelle dans un mélange avec des vecteurs et des excipients.

7. Compositions selon la revendication 6 pour des implants sous-cutanés à administration à libération contrôlée parentérale, intranasale ou orale,
